# EUROPEAN PATENT SPECIFICATION

(11) **EP 0 620 008 B1**
(45) Date of publication and mention of the grant of the patent: **09.06.1999**
(21) Application number: 94103479.5
(22) Date of filing: 08.03.1994
(51) Int. Cl.: A61K 38/00

(54) **Use of vip, analogues and fragments thereof for the treatment of neurodegenerative diseases**
Verwendung von VIP und deren Analogen und Fragmenten zur Behandlung von neurodegenerativen Krankheiten
Utilisation de VIP et ses analogues et fragments pour le traitement des maladies neurodégénératives

(30) Priority: 16.03.1993 IL 10506193
(43) Date of publication of application: 19.10.1994
(73) Proprietor: YEDA RESEARCH AND DEVELOPMENT COMPANY, LTD., Rehovot 76100 (IL); RAMOT UNIVERSITY AUTHORITY FOR APPLIED RESEARCH & INDUSTRIAL DEVELOPMENT LTD., Tel Aviv 69975 (IL)
(72) Inventor: Gozes, Illana, Ramat Hasharon 47445 (IL); Fridkin, Matityahu, Rehovot 76284 (IL)
(74) Representative: VOSSIUS & PARTNER

(56) References cited:
- EP-A- 0 540 969
- WO-A-92/11026
- BRAIN RESEARCH vol. 570, no. 1-2 , 1992 , NEW YORK, NY, USA pages 49 - 53 J. GLOWA ET AL. 'Learning impairment following intracerebral administration of the HIV envelope protein gp120 or a VIP antagonist.'
- PROCEEDINGS OF THE NATIONAL ACADEMY OF SCIENCES OF THE USA vol. 83, no. 4 , February 1986 , WASHINGTON DC, USA pages 1159 - 1162 D. BRENNEMAN ET AL. 'Vasoactive intestinal peptide and electrical activity influence neuronal activity.'
- NATURE vol. 335 , 13 October 1988 , LONDON, GB pages 639 - 642 D. BRENNEMAN ET AL. 'Neuronal cell killing by the envelope protein of HIV and its prevention by vasoactive intestinal peptide.'

## Description

The invention concerns pharmaceutical compositions for the treatment of neurodegenerative diseases, comprising as an active component vasoactive intestinal peptide (VIP) or analogues, derivatives and fragments thereof.

Neurodegenerative diseases in which neuronal cells degenerate bring about a deterioration of cognitive functions. A variety of diseases and neurological deficiencies may bring about the degeneration of neuronal cells among them Alzheimer's disease, Huntington disease or chorea, hypoxia or ischemia caused by stroke, cell death caused by epilepsy, amyotrophic lateral sclerosis, mental retardation etc., as well as neurodegenerative changes resulting from aging.

Vasoactive intestinal peptide (VIP) is a 28 amino acid regulatory peptide which exhibits neurotransmitter and hormonal roles. VIP has a discrete distribution in the central nervous system (CNS), with highest levels in the cerebral cortex, hypothalamus, amygdala and corpus striatum. Accumulated evidence suggest the involvement of VIP in a multitude of neuronal functions among them: changes in membrane potential, modulation of muscarinic excitation, promotion of survival of electrically blocked neurons⁽¹⁾, maintenance of neural integrity⁽²⁾, development of neonatal behavior in rats⁽³⁾, rescue from neural death caused by HIV envelope protein. A VIP-receptor antagonist caused spatial learning impairment in rats which was attenuated by co-treatment with VIP, indicating a possible role for VIP in the process of learning⁽⁴⁾.

There is conflicting evidence concerning the role of VIP in dementia in general and in Alzheimer's disease in particular. A large body of evidence suggests no such involvement. Rosor *et al*. found no changes in VIP in cerebral cortex of brains obtained from patients suffering from senile dementia of the Alzheimer types as compared to controls⁽⁵⁾. Other investigators found no variance in VIP measurement between Alzheimer's patients and controls^{(6,7,8)} and no correlation was found between the degree of dementia and the concentration of VIP in the cerebrospinal fluid⁽⁹⁾.

In contrast, a study of immunoreactivity of VIP in Alzheimer's and control brains showed a significant reduction of VIP immunoreactivity in the cerebral cortex especially in the insular and angulate cortex of Alzheimer's patients⁽¹⁰⁾. However, it has never been determined whether this reduction was the cause or rather the result of the Alzheimer's deterioration of the cerebral cortex.

According to the present invention it has been found that VIP and analogues, derivatives and fragments thereof caused recovery of learning activities in an animal model of Alzheimer.

Thus the present invention provides a pharmaceutical composition for the treatment of neurodegenerative diseases comprising as active ingredient a peptide selected from the group consisting of:
(i) vasoactive intestinal peptide (VIP);
(ii) analogues of vasoactive intestinal peptide (VIP) in which one or more amino acids has been replaced, added or deleted without substantially altering the biological properties of the parent peptide;
(iii) a conjugate being a peptide according to either of (i) and (ii) coupled to a lipophilic moiety;
(iv) a physiologically active fragment of (i), (ii) and (iii); and
(v) a functional derivative of any of (i), (ii), (iii) and (iv);
optionally in combination with a pharmaceutically acceptable carrier.

Said lipophilic moiety which is coupled to the VIP or the modified VIP of Formula I is preferably a saturated or unsatured radical such as hydrocarbyl or carboxylic acyl having at least 5 carbon atoms. The lipophilic moiety can be attached at either or both of the N-terminus and C-terminus of the peptide molecule.

Preferably said analogue of VIP has the sequence of Formula I 
1                              7
His–Ser–Asp–Ala–X¹–Phe–Thr–Asp–Asn–Tyr–Thr–Arg–Leu–Arg     (I)
     16                                                  28
–Lys–Gln–X²–Ala–X³–Lys–Lys–Tyr–Leu–Asn–Ser–Ile–Leu–Asn
 wherein
X¹, X² and X³ may be the same or different and each is a residue of a natural or non-natural lipophilic amino acid.

Most preferably the active agent of the pharmaceutical composition of the invention is selected from a peptide having the sequence of Formula II 
1
R1–Y1–His–Ser–Asp–Ala–X1–Phe–Thr–Asp–Asn–Tyr–Thr (II)
16
–Arg–Leu–Arg–Lys–Gln–X2–Ala–X3–Lys–Lys–Tyr–Leu–Asn
28
–Ser–Ile–Leu–Asn–NH–Y2–R2
 wherein
R¹ and R² may be the same or different and each is hydrogen, a saturated or unsaturated lipophilic group or a C₁-C₄ hydrocarbyl or carboxylic acyl with the proviso that at least one of R¹ and R² is a lipophilic group;
Y¹ and Y² may be the same or different and each is -CH₂- or is a bond in case the associated R¹ or R² is hydrogen and Y¹ may further be -CO-; and
X¹, X² and X³ may be the same or different and each is a residue of a natural or non-natural lipophilic amino acid; or a physiologically active fragment thereof or functional derivatives of said peptide of Formula II or a physiologically active fragment thereof.
X¹, X² and X³ are preferably lipophilic amino acid residues represented by leucine, isoleucine, norleucine, valine, tryptophan, phenylalanine, methionine, octahydroindole-2-carboxylic acid (oic), cyclo-hexylglycine (chg) and cyclopentylglycine (cpg).

Particularly preferred compositions according to the invention comprise peptides of Formula I or II above in which X² is a norleucine residue, X¹ and X³ are each valine, Y¹ is -CO- and R¹ is a C₅ - C₁₇ alkyl with Y¹R¹ being, for example, stearoyl or caproyl, Y² is a bond and R² is hydrogen.

Functional derivatives as described herein are compounds in which at least one of the non-terminal amino acid residues bears a functional group in a side chain, resulting in a biologically active substance. Examples of functional derivatives are glycosides, ethers, esters with both carboxyl and hydroxyl groups, amides, etc.

Analogues of VIP as used herein are peptides in which one or more amino acids has been added, deleted or replaced by any means known in the art. The analogues of VIP which fall under the scope of the present invention are those which maintain the biological properties of native VIP as will be explained hereinbelow.

Physiologically active fragments of the invention as used herein are fragments of VIP, fragments of analogues of VIP and conjugates of a lipophilic moiety with VIP or with analogues or fragments thereof.

The functional derivatives, physiologically active fragments and VIP analogues which fall under the scope of the invention are those which have the activity of protecting electrically blocked neurons from death, or of protecting untreated neurons in culture from naturally occurring death at concentrations which are essentially the same as or lower, as protective concentrations of native VIP, and/or which have the ability to enhance learning and memory acquisition when administered *in vivo* to animals in these concentrations.

The fragments of VIP or of its analogues, are for example, fragments which have the sequence of amino acids in positions 7 to 28 or 16 to 28 of VIP or of the modified VIP of Formula I, or of the conjugate of Formula II. Examples of conjugates of VIP fragments according to the invention are as follows: St-VIP₁₈₋₂₈ (hereinafter *"peptide 6"*) of the formula: St-A-V-K-K-Y-L-N-S-I-L-N; St-Thr⁶-VIP₄₋₁₁ (hereinafter *"peptide 5"*) of the formula: St-A-V-T-T-D-N-Y-T; St-VIP₁₋₁₄ (hereinafter *"peptide 3"*) of the formula: St-H-S-D-A-V-F-T-D-N-Y-T-R-L-R; St-Ala²⁴, Ala²⁵, valine²⁶ VIP₁₅₋₂₇ (hereinafter *"peptide 2"*) of the formula: St-K-Q-M-A-V-K-K-Y-L-A-A-V-L; modified VIP fragments (hereinafter *"peptide 1"*) of the formula: St-H-S-D-G-I-F-T-D-S-Y-S-R-Y-R.

The peptide chains of the peptide compounds of the compositions according to the invention are best prepared by solid phase synthesis^{(11,12)} as known *per se* and once these chains are assembled a terminal group R¹Y¹-and/or R²Y²-that is other than hydrogen, is attached thereto.

When Y¹ is a carbonyl group, i.e. compounds with terminal R¹CO-, the R¹CO- group may be introduced by conventional acylation procedures.

In the preparation of compounds for use in compositions according to the invention in which Y¹ is -CH₂-, i.e. compounds with terminal R¹-CH₂-, the R¹-CH₂-radical may be introduced by first coupling with an aldehyde R¹CH=O and then reducing the resulting R¹-CH=N-grouping by methods known per se. Compounds with terminal R²-CH₂-are obtained by cleaving the peptide with an amine of the formula R²-CH₂-NH₂.

The optional carriers of the pharmaceutical compositions of the invention can be any vehicle for parenteral, oral, aerosol, nasal or ocular administration of drugs acting on the central nervous system. It is preferable to administer a composition according to the invention through the nose, which enables the penetration of the aerosol composition to the CNS through the olfactory nerve (WO 91/07947) or via the ocular route (Chiou, G.C.Y., (1991) *Ann. Rev. Pharmacol. Toxical,* **31**:457-67) or by any other suitable method of administration as described in W.M. Pardridge, *Peptide Drug Delivery*, Raven Press, N.Y., 1991.

The pharmaceutical compositions of the invention may be also directly targeted to the brain by an intercerebroventricular pump.

The present invention further concerns a method of treatment of neurodegenerative diseases by administering to a host in need of such treatment a pharmaceutically effective amount of a peptide as defined in (i) - (v) above.

The present invention still further concerns the use of a peptide as defined in (i) - (v) above for the preparation of a medicament for the treatment of neurodegenerative diseases.

The following examples illustrate the various aspects of the invention it being understood that the invention is not limited thereto.

The following terms used hereinafter have the following connotations:
*VIP* - Vasoactive intestinal agent;
*St-VIP* - Stearoyl VIP;
*Nle*^{*17*}*-VIP* - Norleucine¹⁷-VIP;
*St-Nle*^{*17*}*-VIP* - Stearoyl norleucine¹⁷-VIP.

### BRIEF DESCRIPTION OF DRAWINGS

For better understanding the invention will be described hereinafter, by way of example only, with reference to the annexed drawings in which:
**Fig. 1** shows the effect of varying concentration of VIP (○), St-Nle¹⁷-VIP (▼), Nle¹⁷-VIP (■) and St-VIP (•) on the survival of electrically blocked neurons;
**Fig. 2** shows the effect of various concentrations of peptide 1 on the survival of untreated neurons;
**Fig. 3** shows the effect of various concentrations of peptide 2 on the survival of untreated neurons;
**Fig. 4** shows the effect of various concentrations of peptide 6 on the survival of untreated neurons;
**Figs. 5 A-B** show the effect of various concentrations of peptide 5 on the survival of neurons treated with a VIP antagonist (Fig. 5A) and untreated neurons (Fig. 5B);
**Fig. 6** shows the effect of various concentrations of St-Nle¹⁷-VIP on the survival of neurons treated with fragment 25-35 of β-Amyloid peptide;
**Figs. 7 A-B** show the affinity of St-Nle¹⁷-VIP (Fig. 7A) and St-VIP (Fig. 7B) to the VIP receptor;
**Fig. 8** shows learning and memory studies in control animals (○), animals injected with cholinergic blockers (□) and animals injected with cholinergic blockers and St-Nle¹⁷-VIP (•);
**Fig. 9** shows learning and memory studies in animals injected with cholinergic blocker (□) and animals injected with cholinergic blockers and treated by nasal administration with St-Nle¹⁷-VIP (•), or with peptide 6 (○); and
Fig. 10 shows learning and memory studies in untreated old animals (□) and old animals treated with St-Nle¹⁷-VIP (■).

The following non-limiting examples illustrate the invention.

### EXAMPLE 1: Synthesis of stearoyl-Nle¹⁷-VIP

In an alternative method, synthesis of St-Nle¹⁷-VIP was performed as follows:

The peptide chain was assembled manually in a mechanical shaker according to the general principles of the solid-phase methodology of Merrifield on a 4-(2',4'-dimethoxyphenyl-aminomethyl)-phenoxy resin, purchased from Nova, Switzerland. All solvents: methylene chloride (CH₂Cl₂), N-methylpyrrolidone (NMP), and dimethyl formamide (DMF) were analytical products of Merck. Germany. Trifluoroacetic acid (TFA), diisopropylethylamine (DIEA) and N,N'-dicyclohexylcarbodiimide (DCC) were purchased from Aldrich, U.S.A. 1-Hydroxybenzotriazole (HOBT) was obtained from Nova, Switzerland. All protected amino acid derivatives (FMOC-AA) were of the L-configuration and were obtained from Bachem, Switzerland. N^{α}-amino acid functions were protected throughout the synthesis by the fluorenylmethoxycarbonyl (FMOC) group. Side chain functions were protected as follows: Ser, Asp, Thr with t-butyl; Lys with t-butyloxycarbonyl; His with benzyloxymethyl (BOM); and Arg with methoxytrimethylphenylsulfonyl (Mtr).

The synthesis was initiated by removal of the FMOC-group, from the commercial polymer: 4-(2',4'-dimethoxyphenyl-FMOC-aminoethyl)-phenoxy resin (0.47 mmol of amino group/g), according to steps 1 and 2 (see protocol). 10g of polymer, contained in 2 reaction vessels, were employed. The volume of solvents used was 20-25 ml in each vessel. Assembly of the peptide chain was initiated by coupling FMOC-Asn (0.92g, 4 mmol) to the resin (5g) using DCC (0.84g, 4 mmol) and HOBT (0.55g, 4 mmol) as agents. The coupling was repeated. Loading (0.39 mmol/g) was determined by amino acid analysis. Unreacted residual amino groups on the polymer were capped by reacting with acetic anhydride (10%) and diisopropylethylamine (5%) in CH₂Cl₂. The peptide chain assembly was started from the FMOC-Asn- resin, following the protocol outlined in Table 2.

**TABLE 2**

| **Solid Phase Peptide Synthesis** | | |
|---|---|---|
| Step | Reagents | min. |
| 1 | 10% piperidine/DMF | 5 |
| 2 | 20% piperidine/DMF | 15 |
| 3 | DMF | 2 |
| 4 | DMF | 2 |
| 5 | DMF | 2 |
| 6 | CH₂Cl₂ | 2 |
| 7 | CH₂Cl₂ | 2 |
| 8 | NMP | 2 |
| 9 | Ninhydrin test | |
| 10 | FMOC-amino acid/HOBT/DCC (molar ratio 1:1:1 in NMP preactivation) | |
| 11 | DMF | 2 X 2 |
| 12 | CH₂Cl₂ | 2 |
| 13 | CH₂Cl₂ | 2 |
| 14 | CH₂Cl₂ | 2 |
| 15 | Ninhydrin test | |
| 16 | 10% Ac₂O + 5% DIEA in CH₂Cl₂ | 3 |
| 17 | 10% Ac₂O in CH₂Cl₂ | 5 |
| 18 | CH₂Cl₂ | 2 |
| 19 | CH₂Cl₂ | 2 |
| 20 | CH₂Cl₂ | 2 |
| 21 | DMF | 2 |

Solvents for all washings and reactions were measured to volumes of 10 ml/g resin, except for coupling (step 10) when volumes of about 5 ml/g resin were employed. All couplings were performed using HOBT active esters of FMOC-amino acid derivatives, prepared by DCC prior to each coupling step. A molar ratio of 2:1 of FMOC-amino acid 1-hydroxybenzotriazole ester (FMOC-AA-OBT) and α-amino group of growing peptide chain, respectively, was employed for couplings. Coupling reactions were monitored by boiling a few mg (about 3) of polymer in a solution of ninhydrin in pyridine-water for 2 min. Coupling of FMOC-amino acids was repeated twice or more to ensure complete reaction. In the second, and when necessary other, couplings, half of the amount of FMOC-AA-OBT was used. Proceeding steps, aimed at addition of the next amino acid were initiated only after a negative ninhydrin test (step 15; see protocol). As a rule, after completion of each coupling step, residual amine groups were capped by treating the resin with acetic anhydride (10%) and diisopropylethylamine (5%) in methylene chloride.

Following completion of the peptide chain assembly, the FMOC protecting group of His was removed, as usual, by piperidine in DMF and the newly free α-amino group was coupled (in each reaction vessel) to stearic acid (1.14g, 4 mmol) using DCC (0.84g, 4 mmol) and HOBT (0.54g, 4 mmol) as reagents. The reaction proceeded for 120 min and was repeated twice. The resin containing the fully assembled peptide-chain was washed with CH₂Cl₂ according to protocol, and then dried under vacuum overnight, over P₂O₅. Deblocking of protecting groups and cleavage of the peptide from resin was achieved as follows: 1g of dried resin was placed in a 100 cc flask to which thioanisole (2 ml) and ethanedithiol (2 ml) were added. The mixture was cooled to 4°C in an ice bath and 20 ml of trifluoroacetic acid added, and 5 min later trifluoromethanesulfonic acid (2 ml) was also added. The mixture was gently stirred at room temperature for 50 min.

The reaction mixture was then cooled to 4°C and poured into 500 ml of dry ether. After stirring for 60 min at 4°C, the solid material (resin and peptide) was filtered on a scinter funnel, washed with dry ether, dried and then extracted with 50% aqueous acetic acid (100 ml). The solution obtained, containing the peptide, was concentrated in high vacuum and the residue (about 15 ml) was directly loaded on a Sephadex G25 column (43 x 6 cm). The column was eluted with 0.1N acetic acid at a flow rate of 45 ml/1 hr. Elution was monitored at 274 nm. Lyophilization of the aqueous solution, containing the desired fraction yielded the peptide free of the aromatic additives added as scavengers at the acidolytic cleavage step. Yield was about 400 mg of a white powder.

The material showed the required amino acid content and ratio as revealed by amino acid analysis following exhaustive acid hydrolysis.

Further purification by high performance liquid chromatography (HPLC) was carried out on the Sephadex-fractionated products. It can be performed, however, on the crude peptide. Purifications were achieved on Merck RP-8 column (7µM, 250x25 mm column). The peptide was applied in 35% acetonitrile in water and eluted with a linear gradient established between 35% acetonitrile and 0.1% TEA in water and 0.1% TEA in 75% acetonitrile in water at a flow rate of 10 ml/min. Fractions were collected and cuts made after inspection by analytical HPLC. Derived fractions were pooled and lyophilized. Yield of the pure peptide was 30-35%.

### EXAMPLE 2 - Preparation of R¹-CH₂-Nle¹⁷-VIP derivatives

The peptide chain is assembled on the polymeric support, methyl benzhydryl amine resin (MBHA) (Nova,, Switzerland), (containing 0.39 mmol Asn/1gr) as described in Example 1. After incorporation of the last amino acid residue (histidine) the N-α-protecting group (t-Boc) is removed by TEA, the polymer is treated with DIEA, washed and ninhydrin tested. The polymer is then suspended in ethyl alcohol (1gr/10 ml) and the corresponding aldehyde R'-CH=O is added (3-4 equivalents of aldehyde to 1 equivalent of free N-terminal amino group) and the mixture is gently agitated overnight at room temperature. The polymer is filtered, washed with ethanol (3 x 10 ml), resuspended in ethanol and NaBH₄ (3-4 equivalents of reducing agent to 1 equivalent of Schiff base; R'-CH=N-His···-) and the mixture is gently agitated for 2hr at room temperature. Alternatively, NaBH₃CN (3-4 equivalents to 1 equivalent of Schiff base) can be employed (in the presence of 0.1-0.2 ml of acetic acid). Condensation and reduction reactions can also be performed in other organic solvents, such as DMF or NMP. Following completion of reduction, the polymer is filtered, washed and dried, and treated with HF as described for stearoyl-Nle¹⁷-VIP. The crude product is purified in the same manner as described in Example 1, to afford the desired final products.

### EXAMPLE 3 - Preparation of R¹-Y¹-NH-Nle¹⁷-VIP-NH-R²

The first amino acid, Boc-Asn, was attached to 1% crosslinked chloromethylated polystyrene (Chemalog, South Plainfield, N.J., U.S.A.) as follows: triethylamine (4.75 mmol; 0.66 ml) was added to the amino acid derivative (5 mmole; 1.16 gr) in absolute ethanol (35 ml), and the mixture was allowed to stand for 5 min at room temperature. The polymer (5 gr) was then added and the mixture was gently refluxed for 60 h at 78°C. Alternatively, 5 mmol of Boc-Asn was dissolved in a mixture of EtOH (12 ml) and water (3 ml), and the pH adjusted to 7.5 with a 20% aqueous solution of Cs₂CO₃. The solution was flash evaporated three times with benzene and the residue dried over P₂O₅ in a dessicator for 5h. DMF (30 ml) was then added to dissolve the material, followed by 5 gr of polymer and the mixture was stirred for 36h at 50°C. Loading (0.4 mmol/gr) was determined by amino acid analysis.

Peptide chain assembly was performed as in Example 1. However, Boc-Asp (β-cyclohexyl ester) was used instead of Boc-Asp (β-benzyl ester). The cyclohexyl group is stable toward aminolysis. On completion of the desired peptide chain assembly, the polymer is washed and dried as above. The product is then suspended in absolute ethanol, or a 1:1 v/v mixture of EtOH, and DMF (1 gr/10 ml) and the corresponding amine (R''-NH₂; 20 mmol) is then added and the mixture is gently stirred at room temperature for 48h. TLC, using the solvent system N-butanol : acetic acid : H₂O; pyridine (15 : 3 : 12 : 10 v/v), revealed the appearance of a product which was detached from the polymeric support. The polymer was extracted with ethanol (3 x 10 ml), DMF (3 x 10 ml) and the solvents were evaporated in high vacuum and the oily, semi-solid, residue was then treated, as above, with HF to remove side-chain protecting groups. The crude products were purified in the same manner and comparative yields as described for stearoyl-Nle¹⁷-VIP, to afford the desired final products.

### EXAMPLE 4 Biological tests - effect of VIP and VIP analogues on the survival of electrically blocked cells

### Method

(a) Mouse spinal cord neurons obtained from 12 day old embryos were cultured as described previously⁽¹⁵⁾. The neurons were plated in 10% horse serum 10% fetal calf serum in minimum essential medium eagle (MEM). Medium was changed to 5% horse serum supplemented with nutrients ⁽¹⁵⁾ after a day. Nine days after plating, cultures were given a complete change of medium. All cultures were treated with 1 µM tetrodotoxin (TTX), which is a blocker of electrical activity in neurons, and then divided into four groups. Group 1 was treated with varying concentrations of VIP, group 2 was treated with varying concentrations of Nle¹⁷-VIP, group 3 was treated with varying concentrations of St-VIP, and group 4 was treated with varying amounts of St-Nle¹⁷-VIP. The cultures were treated with VIP or its analogues from day 9 to day 14 and then fixed for immunocytochemistry for neuron specific enolase (NSE), which is a marker of viable neurons. The cells were counted on 100 fields with total area of 50 mm² in a blind test, without knowledge of treatment.

### Results

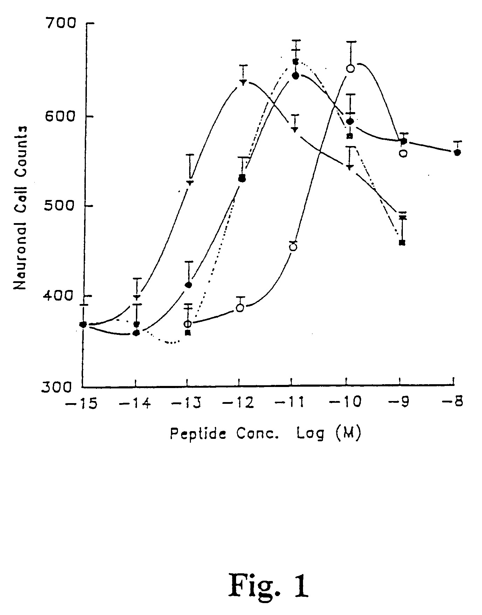

(b) The results are shown in Fig. 1. As can be seen **in Fig. 1**, all active agents of the pharmaceutical compositions of the invention tested protected electrically blocked cells from death to a certain degree, St-VIP and Nle¹⁷-VIP being about 10 fold more potent than native VIP and St-Nle¹⁷-VIP being about 100 fold more potent than native VIP. Both active agents with the hydrophobic moiety (St-VIP and St-Nle¹⁷-VIP) caused also broadening of the peak of active concentrations.

The above results signify that the active agents of the invention are very potent protectors of electrically blocked neurons and thus can serve to decrease neuronal death and subsequent failing in cognitive abilities.

### EXAMPLE 5 Biological test - effects of peptides 1, 2 and 3 on survival of neurons

### Method

(a) Cerebral cortical cultures were prepared by a slight modification of the techniques described by Forsythe and Westbrook, (J. Physiol. Lond. 396:515 (1988)), in which cerebral cortex was used instead of hippocampus and newborn rats were utilized instead of E16 mice. After nine days *growth in vitro*, the cultures were given a complete change of medium and treated with serial dilutions of either peptide 1, 2 or 6 in order to determine whether these peptides were effective in protecting neurons from naturally occurring death. Cerebral cortical cells were counted as above, however, only 40 fields per plate were counted comprising an area of 20 mm².

### Results

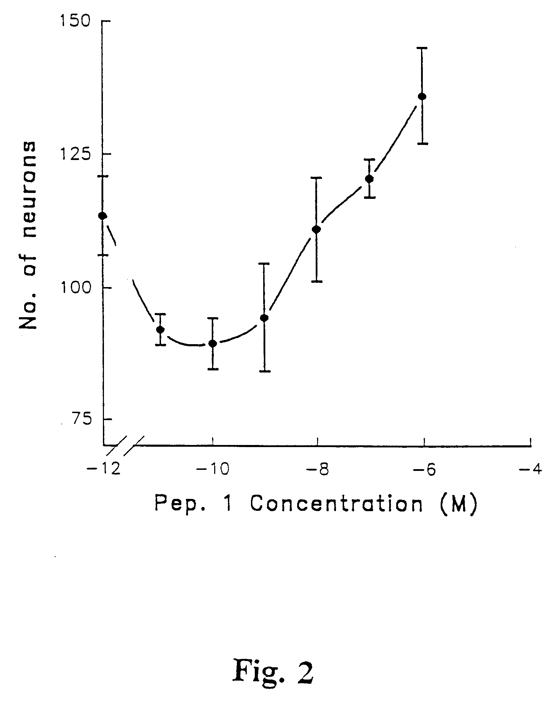

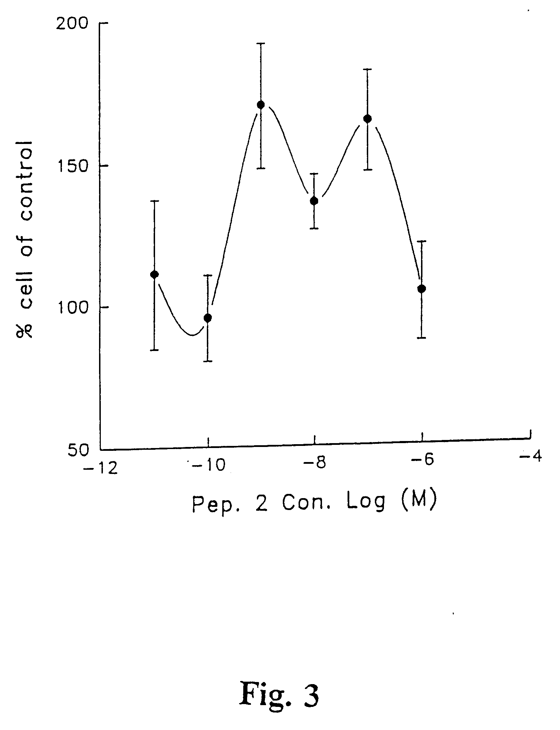

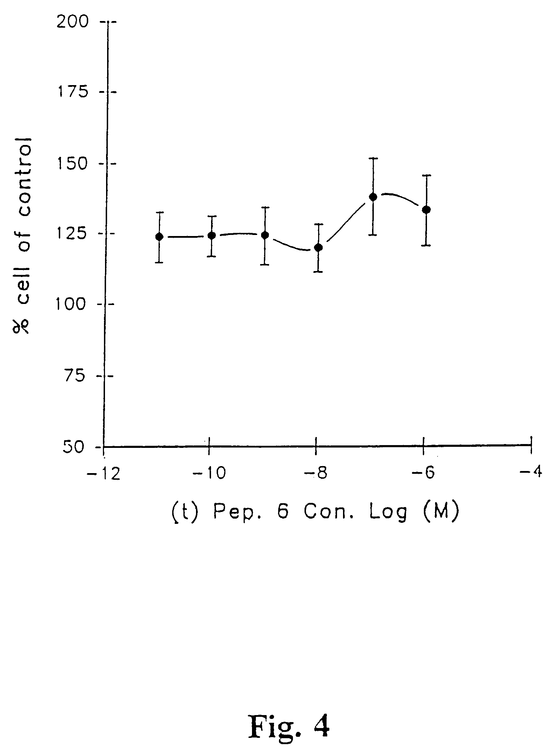

(b) As can be seen in Fig. 2, peptide 1 was able to protect cells from naturally occurring death at concentrations above 10⁻⁸ M (the number of cells at 10⁻¹² M serves as control). Fig. 3 shows that peptide 2 was able to protect cells from death at concentrations above 10⁻⁹ M and Fig. 4 shows that peptide 6 was able to protect cells from natural occurring death at all concentrations tested beginning at 10⁻¹¹ M.

The above results indicate that the active agents of the invention, can protect neurons not only from death caused by electric blockage but also from naturally occurring death.

### EXAMPLE 6 Biological tests - effects of peptide 5 on survival of untreated neurons and neurons treated with a VIP antagonist

### Method

(a) Cells were prepared as described in Example 5. After nine days *growth in vitro*, the cultures were given a complete change of medium and treated with serial dilutions of peptide 5 in the presence and in the absence of 10⁻⁸ M of a VIP antagonist described in detail in U.S. 5,217,953. One treatment was given at the beginning of a five day test period. Cell counts were performed on 100 fields, with a total area of 50 mm². Neurons were counted without knowledge of treatment.

### Results

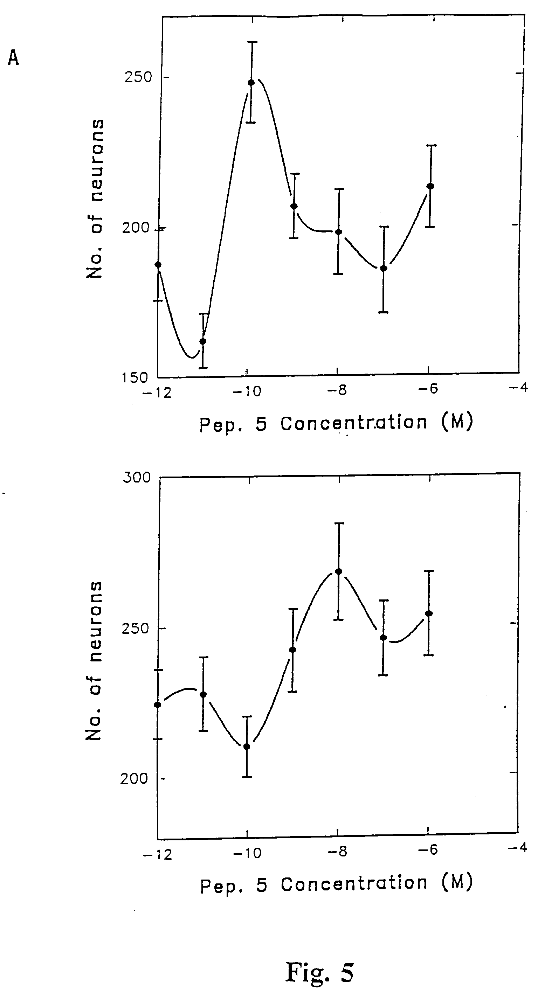

(a) As can be seen in Fig. 5, peptide 5 is a VIP agonist, stimulating neuronal survival in the presence (Fig. 5A) and in the absence (Fig. 5B) of the VIP antagonist indicating that peptide 5 can protect neurons from naturally occurring death. It should be noted that in the presence of the VIP antagonist, the maximal activity is obtained at 10⁻¹⁰ M, while in absence of the VIP antagonist the maximal activity is seen at 10⁻⁸ M, suggesting that the VIP antagonist renders the system more sensitive and enables peptide 5 to exert its protective effects at lower concentrations.

### EXAMPLE 7 Biological tests - effects of St-Nle¹⁷-VIP on survival of neurons treated with fragment 25-35 of β-amyloid peptide

### Method

β-amyloid peptide is known to be involved in Alzheimer's disease and is a toxic substance to neurons grown in culture (Pike *et al., J. of Neurosci*., 13(4), 1676-1687 (1993)). Neurons from rat cerebral cortex, prepared as described above were treated with 25 µM of β-amyloid peptide fragment comprising amino acids in positions 25 to 35 mentioned in the above publication. The β-amyloid peptide fragment was dissolved in water and preincubated at 37°C for 48 h in 10% CO₂. St-Nle¹⁷-VIP (10 µl/1 ml medium) was added to the culture plates at varying concentrations together with the β-amyloid peptide fragment. The St-Nle¹⁷-VIP was initially dissolved in DMSO to give a concentration of 10⁻³ M and all further dilutions were performed in saline. Cells were then incubated for 5 days, stained with antibodies against neuron specific enolase and living neurons were counted as described above.

### Results

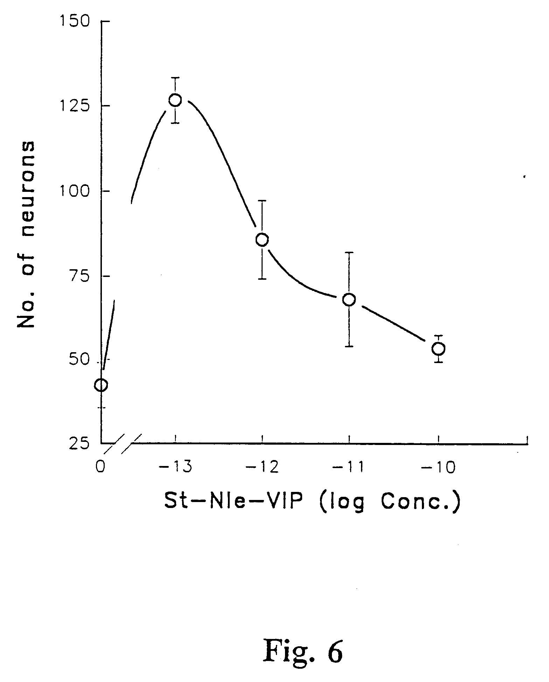

25 µM of fragment 25-35 of β-amyloid peptide alone reduced the number of living neurons to 42 per 20 mm². As can be seen in Fig. 6, St-Nle¹⁷-VIP was able to protect neurons from the toxic effect of β-amyloid peptide fragment at all concentrations tested, the most effective protective concentrations of St-Nle¹⁷-VIP being 10⁻¹³ M while higher concentrations showed a lower protective effect.

These results indicate that the active agents of the invention can protect neurons from death caused by β-amyloid peptide that is involved in Alzheimer's disease neurodegenerative process.

### EXAMPLE 8 Biological test - affinity studies

### Method

(a) Cerebral cortical astrocytes were obtained and cultured as described before^{(16,17)}. Binding studies were performed on intact cells, 5-7 days after replating at 4°C in phosphate buffered saline containing 0.1% bovine serum albumin. Cells were divided into two groups. Group 1 was incubated with varying concentrations of St-VIP and Group 2 was incubated with varying concentrations of St-Nle¹⁷-VIP. After 30 min. incubation with the VIP analogue, 50 pM of ¹²⁵I-VIP⁽¹⁴⁾ was added and incubated for an hour. The media was then removed and cells were washed three times by rapid addition and removal of 1 ml phosphate buffered saline at 4°C. The labeled cells were then dissolved in 0.2 N NaOH and transferred for radioactivity counting by an automatic gamma counter (Wallac 1470 Wizard).

### Results

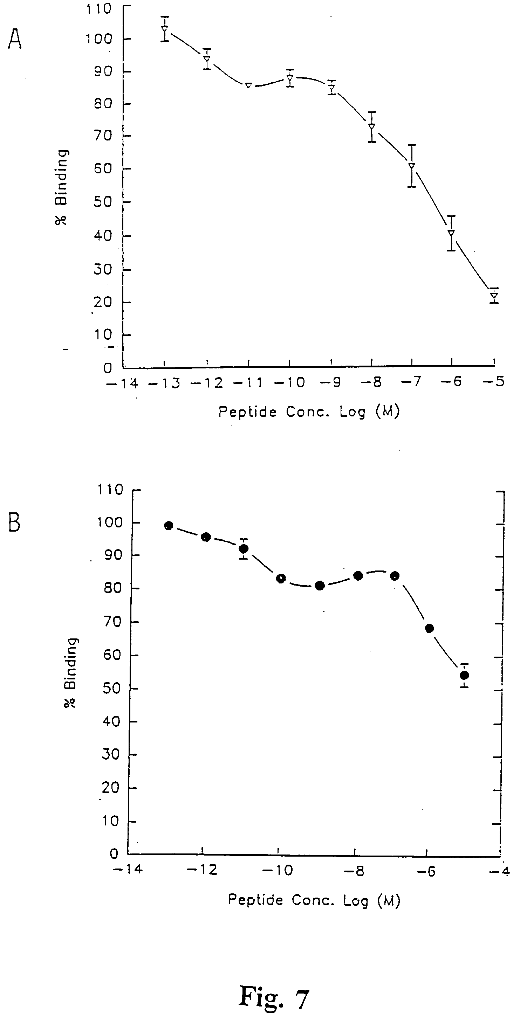

(b) The affinity results are shown in Figs. 7(A) and 7(B). As can be seen at the zone of low concentration (high affinity) St-Nle¹⁷-VIP displaced radiolabeled VIP at concentrations which were 10 fold smaller (Fig. 7(A)) than that of St-VIP (Fig. 7(B)), that is St-Nle¹⁷-VIP has 10 fold higher affinity. At high concentration zone (low affinity zone) St-Nle¹⁷-VIP has also a 10 fold higher affinity than St-VIP. The affinity of St-Nle¹⁷-VIP to the high affinity receptor is about 100 fold higher than that of VIP⁽¹⁹⁾.

### EXAMPLE 9 Biological test - effects of St-Nle¹⁷-VIP on learning and memory in animal models of Alzheimer

### Method

### (a) Preparation of animal models of Alzheimer - I.C.V. drug administration

13 male rats were injected intracerebroventricularly (I.C.V.) at a rate of 0.21 µl/min, using plastic tubing (PE-20) attached to 25G needle. 5 rats serving as control received an injection of saline (2 µl/side), 8 other animals received injections of ethylcholine aziridium (AF64A) (3 nmol/2µl/side). AF64A is an inducer of cholinergic hypofunctions which mimics some of the cholinergic hypofunction reported in Alzheimer's disease and is used for preparing animal models of Alzheimer⁽²²⁾.

Animals were cannulated following injection to allow I.C.V. drug application and left to recover for a week. Learning and memory tests were conducted using the swim maze test. The 8 animals injected with AF64A were divided into two equal groups and were thereafter injected daily with either saline or 100 ng I.C.V. of St-Nle¹⁷-VIP. The five control rats (see above) were injected with saline. Following seven injection days, behavioral assays were conducted for an additional 14 days on both control and test animals.

### (b) Preparation of animal models of Alzheimer-nasal drug administration

8 male rats were all treated with AF64A as described above. 10 days after AF64A administration 5 animals received daily nasal administration of St-Nle¹⁷-VIP dissolved in 10% sefsol and 40% isopropanol at a concentration of 70µg/40µl (20 µl administered through each nostril). 3 animals received daily nasal administration of 50µg/40µl of peptide 6, (20µl/nostril) dissolved in 10% sefsol and 40% isopropanol. 6 control animals received daily nasal administration of 10% sefsol and 40% isopropanol at an amount of 40 µl/day (20µl nostril). The animals were partially anesthetized by diethylether prior to nasal administration. Both control and test animals were daily treated with 50,000 units of durabiotic antibiotics to avoid infection. Following 7 days of nasal administration behavioral tests were conducted on both control and test animals for an additional 8 days.

### Test procedure

Rats were placed in a circular pool, 1.26 m in diameter, 0.2 m deep, equipped with a clear plexiglas column, with a 13.3 cm platform reaching just below the surface of the water (22-24°C). Drugs were applied daily either by injection 4 hours prior to testing or by nasal administration 1 hour prior to testing. The latency of reaching the platform was recorded for each rat (in seconds) and the changes over days of training were graphed, which reflect learning and memory.

### Results

### (a) I.C.V. injected animals

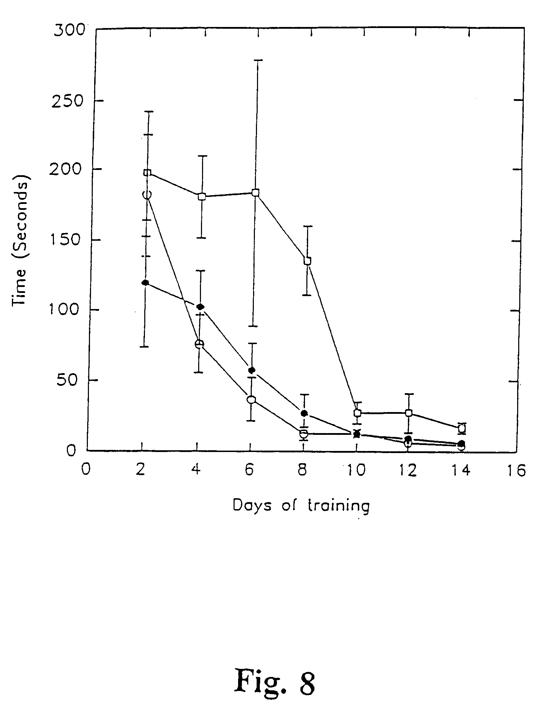

As can be seen in Fig. 8, animals injected with cholinergic blockers (□) showed a smaller improvement in the latency of reaching the platform over time, which indicated a marked decrease in learning and memory as compared to control animals (○). Animals which were injected both with cholinergic blockers and with St-Nle¹⁷-VIP (•) showed recovery of the learning and memory abilities, essentially similar to that of control animals (○).

The above results clearly demonstrate that the agents of the invention are effective in the recovery of learning and memory ability caused by hypofunction of cholinergic neurons, which is a similar condition as appears in Alzheimer.

### (b) Nasal administered animals

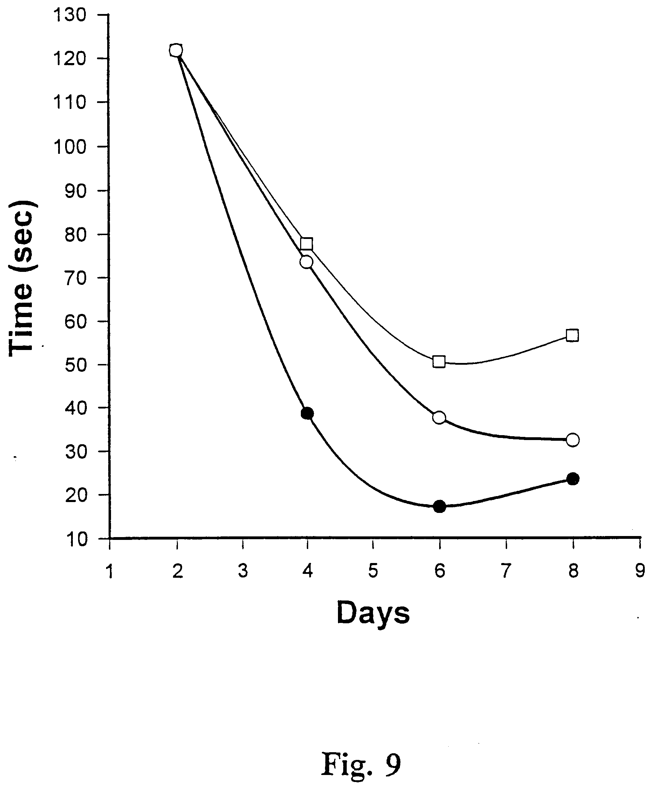

As can be seen in Fig. 9, control animals (□) show smaller improvement in the latency of reaching the platform indicative of a decrease in learning and memory as compared to animals treated with both cholinergic blockers and St-Nle¹⁷-VIP (•) or with cholinergic blockers and peptide 6 (○).

These results clearly indicate that the agents of the invention are also effective when administered by nasal administration.

### EXAMPLE 10 Biological test - effects of St-Nle¹⁷-VIP on learning and memory in old animals

### Method

6 male rats aged 18-20 months were divided into two groups. Group 1 received daily nasal administrations of St-Nle¹⁷-VIP dissolved in 10% sefsol at 40% isopropanol at a concentration of 40 µl/day (20 µl/nostril). Control animals were treated with daily nasal administration of 40 µl/day of 10% sefsol and 40% isopropanol (20 µl/nostril). The administration was carried out after partial anesthetization by diethylether. Animals were thus treated for 7-19 days and then were tested by placing them in a water pool as described above.

### Results

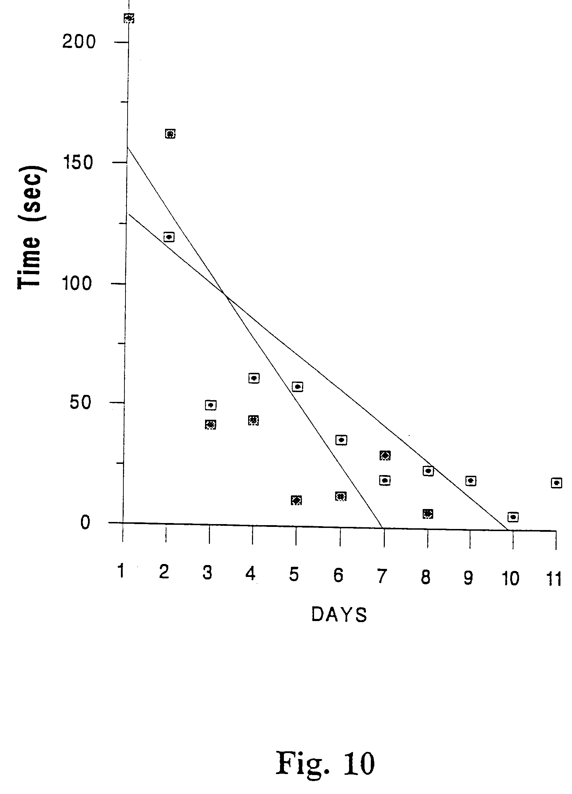

As can be seen in Fig. 10, old animals treated with St-Nle¹⁷-VIP (■) showed a greater improvement in the latency of reaching the platform over time as compared to control untreated animals (•), indicating an increase in learning and memory capacities. These results clearly indicate that the agents of the invention are not only effective in the recovery of learning and memory ability due to pathological conditions such as Alzheimer but also due to normal physiological aging.

### LIST OF REFERENCES

1. Gozes, I. and Brenneman, D.E. (1989) *Molecular Neurobiology*, **3**, 201-236.
2. Gozes, I., Hill, J.M., Mervis, R.F., Fridkin, M. and Brenneman, D.E. (1990) *Soc. Neurosci. Abs.* **16**, 1292.
3. Hill, J.M., Gozes, I., Hill, J.L., Fridkin, M. and Brenneman, D.E. (1991) *Peptides* **12**, 187-192.
4. Glowa, J.R., Panlilio, L.V., Brenneman, D.E., Gozes, I., Fridkin, M., Hill, J.M. (1992) *Brain Res.* **570**, 49-53.
5. Rossor, M. *et al*., (1980) *Brain Res*., **201**, 249-253.
6. Bouras, C., de St. Hilare-Kafi, K. and Constantinidis, J. (1986) *Prog. Neurophychophamacol. Biol. Psychiatry* **10**, 271-286.
7. Sunderland, T. *et al.*, (1991), *Biol. Psychiatry*, **30**(1), 81-87.
8. Sunderland, T. *et al*., (1991), *Biol. Psychiatry*, **30**, 83-87.
9. Wikkelsö *et al*, (1991), *Eur. Neurol*, **31**, 88-93.
10. Arai, H., Moroji, T. and Koska, K. (1984) *Neurosci. Lett.* **52**, 73-78.
11. Merrifield, R.B. (1963) *J. Am. Chem. Soc.* **85**, 2149.
12. Steuart J.M. and Young, J.D., (1984) *Solide Phase Peptide Synthesis*, Pierce Chemical Corp., Rockford, Ill., (2nd edition).
13. Sakakibara, S. *et al*. (1967) *Bull. Chem. Soc. Japan* **40**, 2164.
14. Werner, H. *et al*. (1988) *Biochem. Biophys. Res. Commun.* **133**, 288.
15. Brenneman D.E., Neale, E.A., Foster, G.A., d'Autremont, S., Westbrook, G.L. (1987) *J. Cell Biology* **104**, 1603-1610.
16. McCarthy, K.D. and de Vellis, J. (1980) *J. Cell Biol.* **85**, 890-902.
17. Evans, T., McCarthy, K.D., Harden, T.K. (1984) *J. Neurochem.* **43**, 131-138.
18. Gozes, I., Meltzer, E. Rubinrout, S., Brenneman, D.E. and Fridkin, M. (1989) *Endocrinology* **125**, 2945-2949.
19. Gozes, I., McCune, S.K., Jacobson, L., Warren, D., Moody, T.W., Fridkin, M., Brenneman, D.E. (1991) *J. Pharmacol. Experm. Therap.* **257**, 959-966.
20. Gozes, Y., Brenneman, D.E., Fridkin, M., Asofsky, R., Gozes, I. (1991) *Brain Res.* **540**, 319-321.
21. Tate, B.A., M.K., Lee and C.A. Marotta (1992) *Soc. Neurosci. Abs.* **18**, 197.
22. Fisher, A. *et al.*, (1989), *Neurosci, Letters*, **102**, 325-331.

## Claims

1. Use of a peptide selected from the group consisting of:
(i) vasoactive intestinal peptide (VIP);
(ii) analogues of vasoactive intestinal peptide (VIP) in which one or more amino acids has been replaced, added or deleted without substantially altering the biological properties of the parent peptide;
(iii) a conjugate being a peptide according to either of (i) and (ii) coupled to a lipophilic moiety;
(iv) a physiologically active fragment of (i), (ii) and (iii); and
(v) a functional derivative of any of (i), (ii), (iii) and (iv) for the preparation of a medicament for the treatment of neurodegenerative disease.

2. Use according to Claim 1 in which the active ingredient is an analogue of VIP having the sequence of Formula I 
1                                    7
His-Ser-Asp-Ala-X¹-Phe-Thr-Asp-Asn-Tyr-Thr-Arg-Leu-Arg    (I)
      16                                                    28
-Lys-Gln-X²-Ala-X³-Lys-Lys-Tyr-Leu-Asn-Ser-Ile-Leu-Asn
wherein X¹, X² and X³ may be the same or different and each is a residue of a natural or non-natural lipophilic amino acid.

3. Use according to Claim 2, wherein X¹, X² and X³ are the same or different and each is selected from the group consisting of leucine, isoleucine, norleucine, valine, tryptophan, phenylalanine, methionine, octahydroindole-2-carboxylic acid (oic), cyclohexylglycine (chg) and cyclopentylglycine (cpg).

4. Use according to Claim 1, wherein the peptide is a conjugate of VIP or of an analogue, functional derivative or fragment thereof coupled to a lipophilic moiety at either or both of the N-terminus and C-terminus, wherein said lipophilic moiety is a saturated or unsaturated hydrocarbyl or carboxylic acyl radical having at least 5 carbon atoms.

5. Use according to Claim 1, wherein the peptide has the sequence of Formula II 
1
R¹-Y¹-His-Ser-Asp-Ala-X¹-Phe-Thr-Asp-Asn-Tyr-Thr (II)
16
-Arg-Leu-Arg-Lys-Gln-X²-Ala-X³-Lys-Lys-Tyr-Leu-Asn
28
-Ser-Ile-Leu-Asn-NH-Y²-R²
 wherein
R¹ and R² may be the same or different and each is hydrogen, a lipophilic moiety being a saturated or unsaturated hydrocarbyl or carboxylic acyl having at least 5 carbon atoms, or R¹ and R² may be the same or different and each is a C₁-C₄ hydrocarbyl or carboxylic acyl with the proviso that at least one of R¹ and R² is a C₁-C₄ hydrocarbyl or carboxylic acyl with the proviso that at least one of R¹ and R² is a lipophilic group;
Y¹ and Y² may be the same or different and each is -CH₂ or is a bond in case the associated R¹ or R² is hydrogen and Y¹ may further be -CO-; and
X¹, X² and X³ may be the same or different and each is a residue of a natural or non-natural lipophilic amino acid;
or a physiologically active fragment thereof; and
functional derivatives of said peptide of Formula II or of physiologically active fragments thereof.

6. Use according to Claim 5, wherein X¹, X² and X³ are the same or different and each is selected from the group consisting of leucine, isoleucine, norleucine, valine, tryptophan, phenylalanine, methionine, octahydroindole-2-carboxylic acid (oic), cyclohexylglycine (chg) and cyclopentylglycine (cpg).

7. Use according to Claim 3 or 6, wherein X² is leucine or norleucine and X¹ and X³ are valine.

8. Use according to Claim 5, wherein X² is norleucine, X¹ and X² are valine, Y¹ is -CO-, R¹ is a lipophilic group and R²-Y² is hydrogen.

9. Use according to Claim 8, wherein R¹ is a C₁₇ alkyl.

10. Use according to Claim 1, wherein at least one of the amino acid residues in position 2 to 27 of a peptide of any of groups (i) to (iv) bears a functional group.

11. Use according to Claim 1, wherein the physiologically active fragment has amino acids in position 7 to 28 or 16 to 28 of VIP or of an analogue of VIP.

12. Us according to Claim 1, wherein the peptide is stearoylnorleucine¹⁷-VIP.

13. Use according to Claim 1, wherein the peptide is caproylnorleucine¹⁷-VIP.

14. Use according to Claim 1 for the treatment of dementia.

15. Use according to Claim 14 for the treatment of Alzheimer caused dementia.

16. A conjugate of a lipophilic moiety and a peptide, the conjugate having the following formula
St-A-V-K-K-Y-L-N-S-I-L-N

## Patentansprüche

1. Verwendung eines Peptids, ausgewählt aus:
(i) vasoaktivem intestinalem Peptid (VIP);
(ii) Analogen des vasoaktiven intestinalen Peptids (VIP), in welchen eine oder mehrere Aminosäuren ersetzt, hinzugefügt oder deletiert wurden, ohne die biologischen Eigenschaften des Stammpeptides wesentlich zu ändern;
(iii) einem Konjugat, das ein Peptid nach entweder (i) oder (ii), gekoppelt an eine lipophile Einheit, ist;
(iv) einem physiologisch aktiven Fragment von (i), (ii) und (iii); und
(v) einem funktionellen Derivat nach einem von (i), (ii), (iii) und (iv) zur Herstellung eines Medikamentes zur Behandlung einer neurodegenerativen Krankheit.

2. Verwendung nach Anspruch 1, wobei der aktive Bestandteil ein Analog von VIP ist, das die Sequenz der Formel (I) 
1                    7
His-Ser-Asp-Ala-X¹-Phe-Thr-Asp-Asn-Tyr-Thr-Arg-Leu-Arg     (I)
     16                                                    28
-Lys-Gln-X²-Ala-X³-Lys-Lys-Tyr-Leu-Asn-Ser-Ile-Leu-Asn
 hat,
wobei die Reste X¹, X² und X³ gleich oder unterschiedlich sein können und jeder ein Rest einer natürlichen oder einer nicht-natürlichen lipophilen Aminosäure ist.

3. Verwendung nach Anspruch 2, wobei die Reste X¹, X² und X³ gleich oder unterschiedlich sind und jeder ausgewählt ist aus Leucin, Isoleucin, Norleucin, Valin, Tryptophan, Phenylalanin, Methionin, Octahydroindol-2-carbonsäure (oic), Cyclohexylglycin (chg) und Cyclopentylglycin (cpg).

4. Verwendung nach Anspruch 1, wobei das Peptid ein Konjugat von VIP oder von einem Analog, funktionellen Derivat oder Fragment davon ist, gekuppelt an eine lipophile Einheit, entweder am N-Terminus oder am C-Terminus oder an beiden, wobei die lipophile Einheit ein gesättigter oder ungesättigter Kohlenwasserstoffrest oder ein Carboxylacylrest mit mindestens 5 Kohlenstoffatomen ist.

5. Verwendung nach Anspruch 1, wobei das Peptid die Sequenz der Formel II 
1
R¹-Y¹-His-Ser-Asp-Ala-X¹-Phe-Thr-Asp-Asn-Tyr-Thr (II)
7
16
-Arg-Leu-Arg-Lys-Gln-X²-Ala-X³-Lys-Lys-Tyr-Leu-Asn
28
-Ser-Ile-Leu-Asn-NH-Y²-R²
 hat,
wobei die Reste R¹ und R² gleich oder unterschiedlich sein können und jeder ein Wasserstoffatom oder eine lipophile Einheit bedeutet, die ein gesättigter oder ungesättigter Einheit bedeutet, die ein gesättigter oder ungesättigter Kohlenwasserstoffrest oder ein Carboxylacylrest mit mindestens 5 Kohlenstoffatomen ist, oder R¹ und R² gleich oder unterschiedlich sein können und jeder ein C₁-C₄-Kohlenwasserstoffrest oder ein Carboxylacylrest ist, mit der Maßgabe, daß mindestens einer der Reste R¹ und R² ein C₁-C₄-Kohlenwasserstoffrest oder ein Carboxylacylrest ist, mit der Maßgabe, daß mindestens einer der Reste R¹ und R² eine lipophile Gruppe ist;
Y¹ und Y² gleich oder unterschiedlich sein können und jeweils eine -CH₂-Gruppe oder eine Bindung bedeuten, wenn der assoziierte Rest R¹ oder R² ein Wasserstoffatom ist, und Y¹ ferner -CO- sein kann; und
die Reste X¹, X² und X³ gleich oder unterschiedlich sein können und jeder ein Rest einer natürlichen oder nicht-natürlichen lipophilen Aminosäure ist;
oder ein physiologisch aktives Fragment davon; und
funktioneller Derivate des Peptids von Formel II oder physiologisch aktiver Fragmente davon.

6. Verwendung nach Anspruch 5, wobei die Reste X¹, X² und X³ gleich oder unterschiedlich sind und jeder ausgewählt ist aus Leucin, Isoleucin, Norleucin, Valin, Tryptophan, Phenylalanin, Methionin, Octahydroindol-2-carbonsäure (oic), Cyclohexylglycin (chg) und Cyclopentylglycin (cpg).

7. Verwendung nach Anspruch 3 oder 6, wobei X² Leucin oder Norleucin ist und X¹ und X³ Valin sind.

8. Verwendung nach Anspruch 5, wobei X² Norleucin ist, X¹ und X² Valin sind, Y¹ -CO- ist, R¹ eine lipophile Gruppe ist und R²-Y² Wasserstoff ist.

9. Verwendung nach Anspruch 8, wobei R¹ ein C₁₇-Alkylrest ist.

10. Verwendung nach Anspruch 1, wobei mindestens einer der Aminosäurereste in Position 2 bis 27 des Peptides von einer der Gruppen (i) bis (iv) eine funktionelle Gruppe trägt.

11. Verwendung nach Anspruch 1, wobei das physiologisch aktive Fragment Aminosäuren in Position 7 bis 28 oder 16 bis 28 von VIP oder eines Analogen von VIP hat.

12. Verwendung nach Anspruch 1, wobei das Peptid Stearoylnorleucin¹⁷-VIP ist.

13. Verwendung nach Anspruch 1, wobei das Peptid Caproylnorleucin¹⁷-VIP ist.

14. Verwendung nach Anspruch 1 für die Behandlung von Demenz.

15. Verwendung nach Anspruch 14 für die Behandlung von Alzheimer-bedingter Demenz.

16. Konjugat einer lipophilen Einheit und eines Peptids, wobei das Konjugat die folgende Formel hat:
St-A-V-K-K-Y-L-N-S-I-L-N.

## Revendications

1. Utilisation d'un peptide choisi parmi le groupe consistant en :
(i) le peptide intestinal vasoactif (VIP) ;
(ii) les analogues du peptide intestinal vasoactif (VIP) dans lequel un ou plusieurs acides aminés a été remplacé, ajouté ou délété sans altération substantielle des propriétés biologiques du peptide parent ;
(iii) un conjugué qui est un peptide selon l'un de (i) et (ii) coupé a un reste lipophile ;
(iv) un fragment physiologiquement actif de (i), (ii) et (iii); et
(v) un dérivé fonctionnel de l'un quelconque de (i), (ii), (iii) et (iv) pour la préparation d'un médicament pour le traitement de maladies neurodégénératives.

2. Utilisation selon la revendication 1 dans laquelle le principe actif est un analogue du VIP ayant la séquence de formule I 
1                              7
His-Ser-Asp-Ala-X¹-Phe-Thr-Asp-Asn-Tyr-Thr-Arg-Leu-Arg     (I)
      16                                                    28
-Lys-Gln-X²-Ala-X³-Lys-Lys-Tyr-Leu-Asn-Ser-Ile-Leu-Asn .
dans laquelle X¹, X² et X³ peuvent être identiques ou différents et sont chacun un résidu d'un acide aminé lipophile naturel ou non naturel.

3. Utilisation selon la revendication 2, dans laquelle X¹, X² et X³ sont identiques ou différents et sont chacun choisis parmi le groupe consistant en leucine, isoleucine, norleucine, valine, tryptophane, phénylalanine, méthionine, acide octahydroindole-2-carboxylique (oic), cyclo-hexylglycine (chg) et cyclopentylglycine (cpg).

4. Utilisation selon la revendication 1, dans laquelle le peptide est un conjugué du VIP ou d'un analogue, dérivé fonctionne ou fragment de celui-ci, coupé à un reste lipophile à l'une ou aux deux extrémités N-terminale et C-terminale, dans laquelle ledit reste lipophile est un radical hydrocarbyle ou acyle carboxylique saturé ou insaturé ayant au moins 5 atomes de carbone.

5. Utilisation selon la revendication 1, dans laquelle le peptide a la séquence de formule II 
1
R¹-Y¹-His-Ser-Asp-Ala-X¹-Phe-Thr-Asp-Asn-Tyr-Thr (II)
16
-Arg-Leu-Arg-Lys-Gln-X²-Ala-X³-Lys-Lys-Tyr-Leu-Asn
28
-Ser-Ile-Leu-Asn-NH-Y²-R²
 dans laquelle :
R¹ et R² peuvent être identiques ou différents et sont chacun un hydrogène, un reste lipophile qui est un reste hydrocarbyle ou acyle carboxylique saturé ou insaturé ayant au moins 5 atomes de carbone, ou R¹ et R² peuvent être identiques ou différents et être chacun un reste hydrocarbyle ou acyle carboxylique en C₁-C₄, sous réserve qu'au moins un de R¹ et R² est un reste hydrocarbyle ou acyle carboxylique en C₁-C₄ avec la réserve que l'un au moins de R¹ et R² est un groupe lipophile;
Y¹ et Y² peuvent être identiques ou différents et être chacun -CH₂ ou une liaison dans le cas où R¹ ou R² qui sont associés sont un hydrogène et Y¹ peut en outre être -CO-; et
X¹, X² et X³ peuvent être identiques ou différents et être chacun un reste d'un acide aminé lipophile naturel ou non naturel;
ou un fragment physiologiquement actif de celui-ci; et
les dérivés fonctionnels dudit peptide de formule II ou de fragments physiologiquement actifs de celui-ci.

6. Utilisation selon la revendication 5, dans laquelle X¹, X² et X³ sont identiques ou différents et sont chacun choisis dans le groupe consistant en leucine, isoleucine, norleucine, valine, tryptophane, phénylalanine, méthionine, acide octahydroindole-2-carboxylique (oic), cyclo-hexylglycine (chg) et cyclopentylglycine (cpg).

7. Utilisation selon la revendication 3 ou 6, dans laquelle X² est la leucine ou la norleucine et X¹ et X³ sont la valine.

8. Utilisation selon la revendication 5, dans laquelle X² est la neuroleucine, X¹ et X² sont la valine, Y¹ est -CO-, R¹ est un groupe lipophile et R²-Y² est l'hydrogène.

9. Utilisation selon la revendication 8, dans laquelle R¹ est un alkyle en C₁₇.

10. Utilisation selon la revendication 1, dans laquelle au moins un des restes d'acide aminé en position 2 à 27 du peptide de l'un quelconque des groupes (i) à (iv) porte un groupe fonctionnel.

11. Utilisation selon la revendication 1, dans laquelle le fragment physiologiquement actif possède les acides aminés en position 7 à 28 ou 16 à 28 du VIP ou d'un analogue du VIP.

12. Utilisation selon la revendication 1, dans laquelle le peptide est le stéaroyl-norleucine¹⁷-VIP.

13. Utilisation selon la revendication 1, dans laquelle le peptide est le caproyl-norleucine¹⁷-VIP.

14. Utilisation selon la revendication 1 pour le traitement de la démence.

15. Utilisation selon la revendication 14 pour le traitement de la démence causée par la maladie d'Alzheimer.

16. Conjugué du reste lipophile et d'un peptide, le conjugué ayant la formule suivante :
St-A-V-K-K-Y-L-N-S-I-L-N.
